# EUROPEAN PATENT APPLICATION

(11) **EP 2 695 889 A1**
(43) Date of publication of application: **12.02.2014**
(21) Application number: 13191332.9
(22) Date of filing: 28.12.2010
(51) Int. Cl.: C07K 1/18, C07K 16/00, C07K 16/06, C07K 16/36

(54) **Protein purification by ion exchange**

(30) Priority: 29.12.2009 IN CH32132009
(62) Divisional of application: 10844264.1
(71) Applicant: Dr. Reddy's Laboratories Limited, Hyderabad 500 016, Andhra Pradesh (IN); Dr. Reddy's Laboratories Inc., Bridgewater, NJ 08807 (US)
(72) Inventor: Kulkarni, Samir, 400 072 Andheri East Maharashtra (IN); Subrahmanyam, Satyam, 500 028 Hyderabad Andhra Pradesh (IN)
(74) Representative: Bates, Philip Ian

(57) **Abstract**

A process for purifying antibodies, comprising:
a) purifying using protein A chromatography, wherein an antibody is eluted at a first pH value;
b) purifying using cation exchange chromatography performed in the bind-elute mode, wherein eluate obtained from step a) is loaded onto a cation exchange resin at a second pH value, and elution is carried out at a third pH value; and
c) purifying using anion exchange chromatography performed in the flow-through mode, wherein eluate from step b) is loaded on to an anion exchange resin without substantial adjustment of pH.

## Description

### INTRODUCTION

Aspects of the application relate to methods of purifying antibodies using chromatographic methods. In embodiments, the application describes antibody purification methods comprising multiple chromatographic steps, wherein a low pH eluate from a protein A chromatography is further purified without a requirement for substantial pH adjustment.

Large scale purification of proteins remains a significant challenge in the biopharmaceutical industry as efficient and cost-effective methods are required to achieve desired yields and purity levels. Therapeutic proteins are primarily produced by recombinant DNA technology, i.e., by cloning and expression of a heterologus gene in prokaryotic or eukaryotic systems. However, proteins expressed by recombinant DNA methods are typically associated with contaminants such as host cell proteins ("HCP"), host cell DNA ("HCD"), viruses, etc. In addition, there is significant microheterogeneity in the expression of the desired protein, in the form of charged variants (typically acidic, lower pl variants and basic, higher pl variants).The presence of these contaminants, including undesirable charged variants, are a potential health risk, and hence their removal from a final product is a regulatory requirement. Thus, drug regulatory agencies such as United States Food and Drug administration ("FDA") require that biopharmaceuticals be free from impurities, both product related (aggregates or degradation products) and process related (media components, HCP, DNA, chromatographic media used in purification, endotoxins, viruses, etc). See, *Office* of *Biologics Research and Review, Food and Drug Administration, Points to consider in the production and testing of new drugs and biologicals produced by recombinant DNA technology (Draft),* 1985. Thus, elimination of impurities and contaminants from a final product is mandatory and poses a significant challenge in the development of methods for the purification of proteins.

Protein purification is usually a multistep process, wherein different chromatographic steps are run sequentially to yield a final purified product. For purification of monoclonal antibodies, protein A chromatography is one of the more widely used methods, and usually is the first step in antibody purification. This is a type of affinity chromatography wherein separation is affected by means of a resin tagged with protein A (Hjelm H. et.al., FEBS lett. 1972; 28, 73-76; Langone JJ., Adv Immunol, 1982; 32, 157-252). The various aspects of Protein A chromatography (protein A and its variants, chromatographic medium, etc.) are described in U.S. Patent Nos. 6,013,763 and 6,399,750, and European Patent Application Publication Nos. 282308 and 284368.

A disadvantage of protein A chromatography is the leaching of Protein A and its fragments from the chromatographic resin and its contamination of the eluate. Since protein A is of bacterial origin (obtained from *Staphylococcus aureus),* it's removal is necessary to avoid undesirable immune responses. It has been shown that IgG can form complexes with protein A that may activate Fc bearing leukocytes and complement system to generate oxidant and anaphylatoxin activity *in vitro* (Balint J. et.al., Cancer Res. 1984; 44, 734-743). Further, protein A has also been linked with toxicity (Bensinger, W.I. et. al., J. Biol Resp. Modif. 1984; 3, 347; Messeschimdt GL. et. al., J. Biol. Resp. Modif. 1984; 3, 325; Terman D.S. and Bertram, J.H., Eur. J. Cancer Clin. Oncol. 1985; 21, 1115 and Ventura G.J et. al., Cancer Treat. Rep. 1987; 71, 411). Thus, subsequent purification steps are required to remove protein A leachates, as well as residual host cell proteins, host cell DNA, etc. to meet regulatory requirements.

The literature discloses various methods for purification of crude or partially purified samples. Balint et. al. describe the use of gel filtration for separating uncomplexed antibodies from IgG-protein A complexes (Balint et.al., Cancer Res 1984; 44, 734-743). U.S. Patent No. 4,983,722, European Patent Application Publication No. 1601697 and U.S. Patent Application Publication No. 2007/0292442 describe the use of ion exchange chromatography for purification of antibodies. However, these methods either result in considerable loss of antibody or require substantial pH adjustment of the sample prior to a subsequent chromatography step. This change in pH is achieved by addition of a high molarity base that compromises process efficiency as a result of volume dilution and mixing efficiency, as well as product stability due to localized pH surge. The impact on product stability is of particular significance as it leads to significant product loss due to denaturation, precipitation, and aggregation.

There remains a need for efficient multi-step separation processes for antibodies.

### SUMMARY

In aspects, the application describes antibody purification methods, comprising multiple chromatographic steps wherein a low pH eluate from a protein A chromatography is further purified without the need of substantial pH adjustment.

### BRIEF DESCRIPTION OF THE DRAWINGS

Fig. 1 is an illustration of a chromatogram from the procedure of Example 1.
Fig. 2 is an illustration of a chromatogram from the procedure of Example 2.
Fig. 3 is an illustration of a chromatogram from the procedure of Example 5.
Fig. 4 is an illustration of a chromatogram from the procedure of Example 3.
Fig. 5 is an illustration of a chromatogram from the procedure of Example 5.
Fig. 6 is an illustration of a chromatogram from the procedure of Example 5.

### DETAILED DESCRIPTION

The term "flow-through mode" as used herein refers to chromatographic methods wherein a desired protein is obtained in the flow-through liquid during loading or post-loading washing of a chromatography column. A desired protein in the flow-through liquid may be collected as various fractions and pooled together or can be collected as a single fraction.

The term "bind-elute mode" as used herein refers chromatographic methods wherein a desired protein is bound to the chromatography resin when loaded onto a column and is eluted subsequently using an elution buffer. The desired protein is collected in an elution liquid and may be collected as a single fraction or as various fractions that are pooled together.

The term "antibody" as used herein refers to an immunoglobulin that is composed of four polypeptide chains consisting of two light and two heavy chains, as well as immunoglobulins isolated from various sources, such as murine, human, recombinant, etc., truncated antibodies, chimeric, humanized, or pegylated antibody isotypes, allotypes, and alleles of immunoglobulin genes. The term antibody also refers to fusion proteins that contain an immunoglobulin moiety.

In the purification of monoclonal antibodies ("MAbs"), protein A chromatography is a commonly used method, as highly purified MAbs can be obtained due to the high specificity and binding between a protein A ligand and a Fc region of the antibody. However, as discussed earlier, a disadvantage of protein A chromatography is the leaching of protein A and its fragments into the eluate. Hence, further purification steps are required for the removal of protein A and/or its fragments, as well as residual host cell proteins, endotoxins, and host cell DNA.

U.S. Patent No. 4,983,722 and European Patent Application Publication No. 1601697 describe the use of anion exchange chromatography for the purification of antibodies. However, the anion exchange step is performed at neutral to alkaline pH values, necessitating substantial pH adjustment of the antibody sample. For instance, in US 4,983,722 the protein A eluate is diafiltered against a DEAE equilibration buffer at pH 8.6, while in EP 1601697 the acidic protein A eluate is neutralized with a high molarity buffer, such as 0.5M TrisHCl pH 7.5, and diafiltered with binding/equilibration buffer at pH 8.0 prior to the next chromatographic step. Likewise, U.S. Patent Application Publication No. 2007/0292442 describes the use of two ion exchange resins for the purification of antibodies, wherein the pH of the first eluate is adjusted before loading onto the second ion exchange resin. The pH adjustment greatly compromises both the process efficiency and the product stability. Hence a process involving no, or minimal, pH adjustment will be a better alternative to the current methods.

In aspects, the present application describes processes that reduce the need of pH adjustment in a multistep purification process, thereby increasing process efficiency and product stability, in addition to achieving effective separation of acidic and basic variants of monoclonal antibodies.

In an aspect, the application provides methods for purifying antibodies, embodiments comprising:
1) A first purification step using protein A chromatography, wherein an antibody is eluted at particular pH values.
2) A second purification step using cation exchange chromatography that is performed in the bind-elute mode, wherein eluate obtained from step 1) is loaded onto a cation exchange resin without substantial adjustment of pH (viz. within a range of ± 0.2 pH values).

In embodiments, an antibody is eluted in purification step 1) at pH values about 3.5 to 6 and loaded onto a cation exchange resin at pH values about 3.5 to 6.

In embodiments, an antibody is eluted in purification step 1) at pH values about 3.5 and loaded onto a cation exchange resin at pH values about 3.5.

In an aspect, the application provides methods for purifying antibodies, embodiments comprising:
1) A first purification step using protein A chromatography, wherein an antibody is eluted at a first pH.
2) A second purification step using cation exchange chromatography performed in the bind-elute mode, wherein eluate obtained from step 1) is loaded onto a cation exchange resin at a second pH, and the elution is carried out at a third pH.
3) A third purification step using anion exchange chromatography performed in the flow-through mode, wherein eluate from step 2) is loaded onto an anion exchange resin without substantial adjustment of pH (viz. within a range of ± 0.2 pH values).

In embodiments, the first, second, and third pH values may be same or different.

In embodiments, the first and second pH values may be similar.

In embodiments, the first and second pH values may be different.

The protein A chromatographic resin used may be any protein A or variant or a functional fragment thereof coupled to any chromatographic support. In embodiments, the protein A resin is Prosep vA Ultra^{®} (from Millipore), wherein animal-free protein A is immobilized on porous glass. In embodiments, fresh (i.e., not previously used) protein A chromatographic resin may be used to obtain a feed stream for a second chromatographic step. After washing with loading buffer and intermediate wash, the elution is carried out at low pH such as from pH 2.5 to about pH 4.5

Cation exchange chromatographic step mentioned in the embodiments may be carried out using any weak or strong cation exchange chromatographic resin or a membrane which could function as a weak or a strong cation exchanger. Commercially available cation exchange resins include, but are not limited to, those having a sulfonate based group e.g., MonoS, MiniS, Source 15S and 30S, SP Sepharose Fast Flow, SP Sepharose High Performance from GE Healthcare, Toyopearl SP-650S and SP-650M from Tosoh, S-Ceramic Hyper D, from Pall Corporation or a carboxymethyl based group e.g., CM Sepharose Fast Flow from GE Healthcare, Macro-Prep CM from BioRad, CM-Ceramic Hyper D, from Pall Corporation, Toyopearl CM-650S, CM-650M and CM-650C from Tosoh. In embodiments of the invention, a weak cation exchange resin, such as CM Ceramic Hyper D F^{®} (Pall Corporation) is used; this is made using rigid porous beads that are coated with functionalized hydrogel.

Anion exchange chromatography mentioned in the embodiments may be carried out using any weak or strong anion exchange chromatographic resin or a membrane which could function as a weak or a strong anion exchanger. Commercially available anion exchange resins include, but are not limited to, DEAE cellulose, Poros PI 20, PI 50, HQ 10, HQ 20, HQ 50, D 50 from Applied Biosystems, MonoQ, MiniQ, Source 15Q and 3OQ, Q, DEAE and ANX Sepharose Fast Flow, Q Sepharose high Performance, QAE SEPHADEX and FAST Q SEPHAROSE from GE Healthcare, Macro-Prep DEAE and Macro-Prep High Q from Biorad, Q-Ceramic Hyper D, DEAE-Ceramic Hyper D, from Pall Corporation. In embodiments of the invention, a strong anion exchange resin, such as Q-Sepharose Fast Flow^{®} (GE Healthcare Life Sciences) is used. This resin is made using a highly cross-linked, 6 % agarose matrix attached to -O-CH₂CHOHCH₂OCH₂CHOHCH₂N⁺(CH₃)₃ functional group.

Examples of buffering agents used in the buffer solutions include, but are not limited to, TRIS, phosphate, citrate, and acetate salts, or derivatives thereof.

Protein A leachates can be analysed using protein A ELISA and purified antibodies can be analysed using protein A high performance liquid chromatography.

Certain specific aspects and embodiments of the application are more fully described by reference to the following examples, being provided only for purposes of illustration. These examples should not be construed as limiting the scope of the application in any manner.

### EXAMPLE 1

### Protein A chromatography

An anti-VEGF antibody was cloned and expressed in a CHO cell line as described in U.S. Patent No. 7,060,269, which is incorporated herein by reference. The cell culture broth containing the expressed antibody was harvested, clarified and subjected to protein A affinity chromatography as described below.

The clarified cell culture broth was loaded onto a protein A chromatography column (Prosep vA ultra, VL44x250, 205 mL) that was pre-equilibrated with 5 column volumes ("CV") of 50 mM Tris, 150 mM NaCl, pH 7.5 buffer. The column was then washed with 5 CV of the equilibration buffer (50 mM Tris, 150 mM NaCl, pH 7.5). This was followed by a wash with 5 CV of 50 mM Tris, 750 mM NaCl, pH 7.5 buffer and a final wash with 25 mM Tris at pH 7.5. The bound antibody was eluted using the low pH buffer 200 mM acetate buffer, pH 3.5.

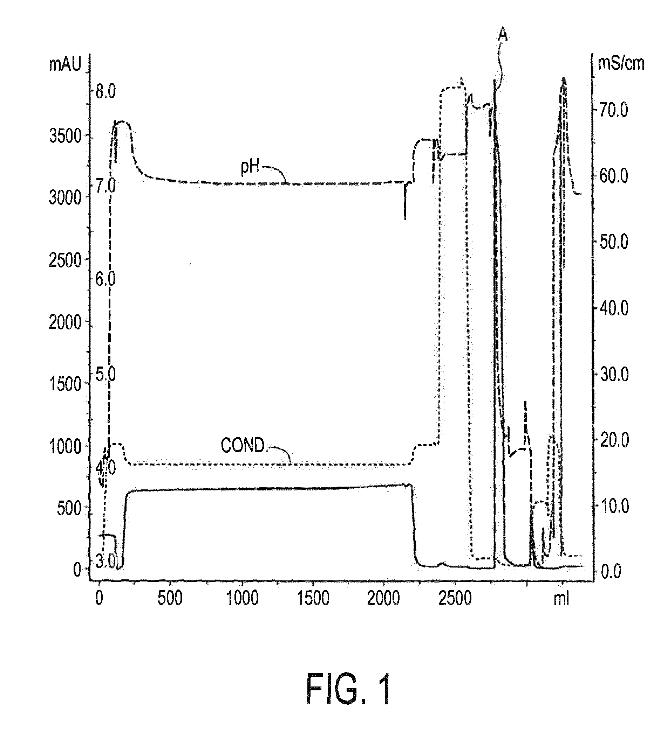

Figure 1 is an illustration of a chromatogram from the procedure as described in this example. The line marked "Cond" represents the increase in conductivity in mS/cm. Peak A, represents the eluate obtained from protein A chromatography resin.

### EXAMPLE 2

### Cation exchange chromatography

The eluate obtained from the protein A chromatography procedure described in Example 1 was loaded onto a cation exchange resin (CM Ceramic Hyper D F, VL44x250, 304 mL) pre-equilibrated with 10 CV of equilibration buffer (200 mM acetate buffer, pH 3.5). This was followed by washing with 30 CV of wash buffer (35 mM Phosphate buffer pH 6.0). The bound antibody was eluted using a conductivity gradient (2.5 mS/cm to- 7 mS/cm) with a phosphate buffer (35 mM to 80 mM, pH 6.0).

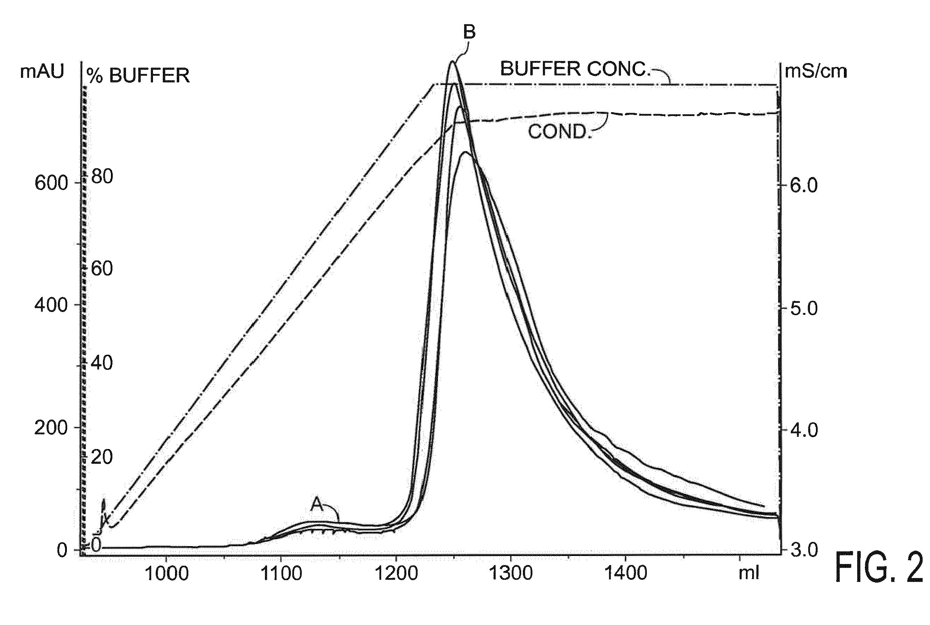

Figure 2 is an illustration of a chromatogram from the procedure as described in this example. The line marked "Cond" represents the increase in conductivity in mS/cm. "Buffer Conc." represents the concentration of phosphate buffer during the chromatographic run, where 100% corresponds to a buffer concentration of 80 mM. Peak A represents the acidic variant of the antibody, and Peak B represents the more basic (desired) variant of the antibody. The consistency of elution (Peak B) in multiple runs is shown.

### EXAMPLE 3

### Anion exchange chromatography

The eluate obtained from the cation exchange chromatography procedure described in Example 2 was loaded onto an anion exchange resin (Q-Sepharose FF, VL32x250, 80 mL) pre-equilibrated with 5-20 CV of an equilibration buffer (67 mM phosphate buffer, pH 6). This was followed by a post load wash with 5 CV of equilibration buffer and the load and wash flow-through was collected.

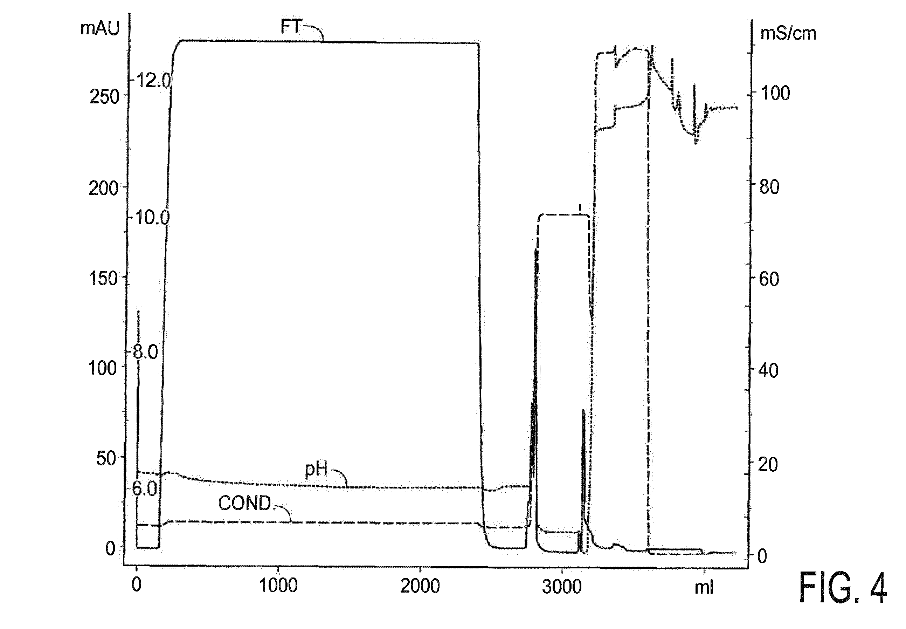

Figure 4 is an illustration of a chromatogram from the procedure described in this example. The line marked "Cond" represents the increase in conductivity in mS/cm. "FT" represents the flow-through obtained. Impurity levels in the cation exchange chromatography eluate and anion exchange chromatography flow through are described in Table 1.

**Table 1**

| **Sample** | **Impurity Concentration** | | | **Step Recovery (%)** | **Monomer (%)** |
|---|---|---|---|---|---|
| | **Host Cell Proteins (ppm)** | **Host Cell DNA (ng/mL)** | **Protein A leachate (ppm)** | | |
| Clarified cell culture broth | 50170 | 296 | - | 100 | |
| Protein A eluate (eluate pH 3.5) | 5.0 | ND | 23.3 | 97 | |
| Cation Exchange chromatography eluate (load pH 3.5) | BDL | <0.19 | BDL | 53 | 99.8 |
| Anion Exchange chromatography flow through (load pH 6.0) | BDL | <0.19 | BDL | 97 | |

| | | | | | |
|---|---|---|---|---|---|
| BDL: Below detection limit. ppm: parts per million. ND: not determined. | | | | | |

### EXAMPLE 4

### Protein A chromatography

Clarified cell culture broth from Example 1 was loaded onto a protein A chromatography resin (Prosep vA ultra, VL44x250, 205 mL) that was pre-equilibrated with 5 CV of a 50 mM Tris, 150 mM NaCl, pH 7.5 buffer. The column was then washed with 5 CV of an equilibration buffer (50 mM Tris, 150 mM NaCl, pH 7.5). This was followed by a wash with 5 CV of a 50 mM Tris, 750 mM NaCl, pH 7.5 buffer and a final wash with 25 mM Tris at pH 7.5. The bound antibody was eluted using the low pH buffer 200 mM acetate buffer, pH 3.5.

### EXAMPLE 5

### Cation exchange chromatography

The eluate obtained from protein A chromatography step described in Example 4 was adjusted to pH 6.0 and loaded onto a cation exchange resin (CM-Ceramic Hyper D F, VL44x250, 304 mL) pre-equilibrated with 10 CV of equilibration buffer (35 mM phosphate buffer pH 6.0). This was followed by washing with 10 CV of equilibration buffer. The bound antibody was eluted using a conductivity gradient (2.5 mS/cm to 7 mS/cm) with a phosphate buffer (35 mM to 80 mM, pH 6.0). Alternatively, elution was done using a step-gradient with less than 3 to 5 CV of phosphate buffer, pH 6.2 wherein the concentration of the buffer was increased in steps from 35 mM to 80 mM, or from 35 mM to 90 mM.

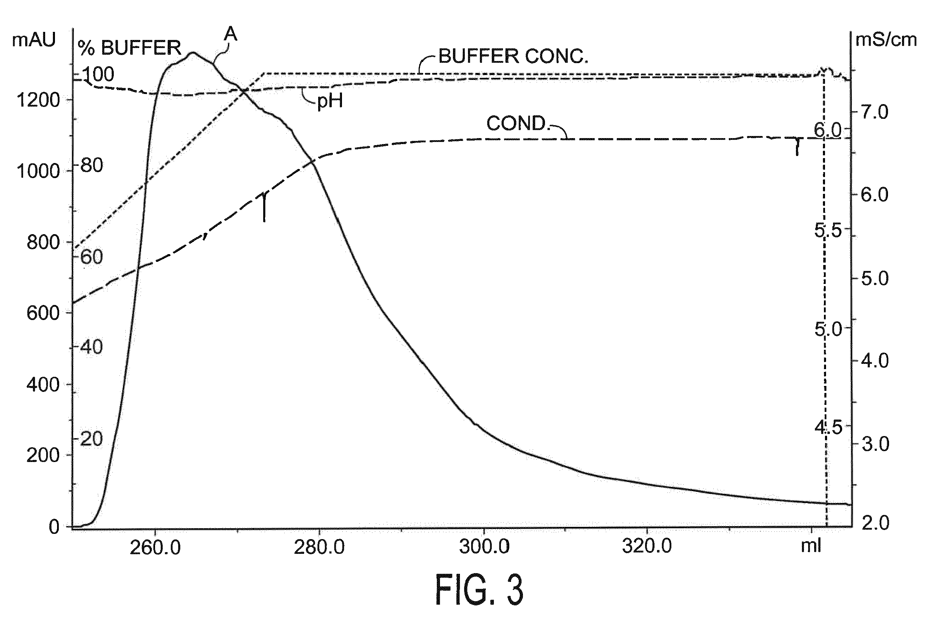

Figure 3 is an illustration of a chromatogram from the procedure described in this example. The line marked "Cond" represents the increase in conductivity in mS/cm. "Buffer Conc." represents the concentration of phosphate buffer during the chromatographic run, where 100% corresponds to a buffer concentration of 80 mM. Peak A represents the eluate obtained from the linear increase in conductivity.

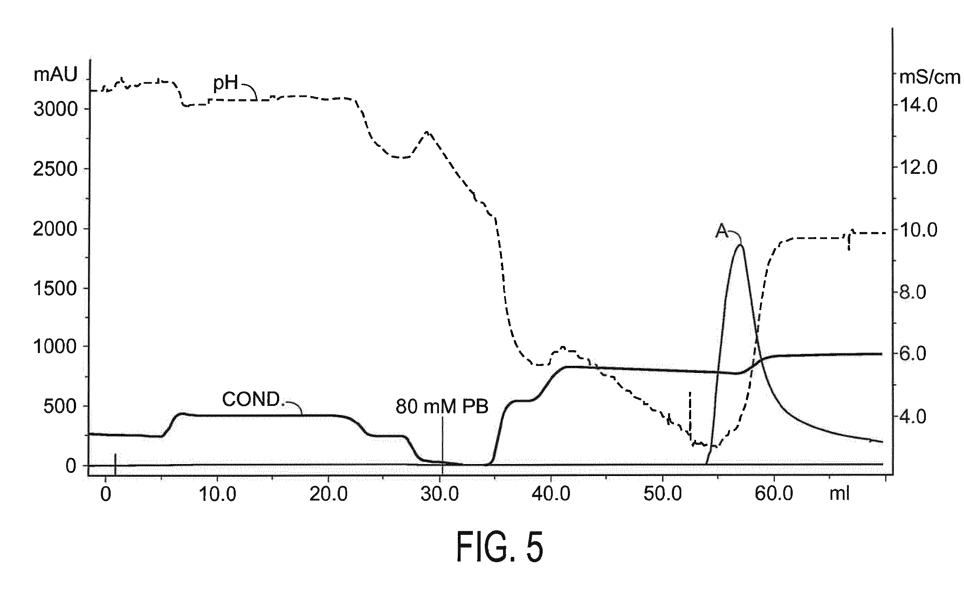

Figure 5 is an illustration of a chromatogram from the procedure described in this example. The line marked "Cond" represents the increase in conductivity in mS/cm. Peak A represents the eluate obtained from the step increase in conductivity from 35 mM buffer to 80 mM buffer.

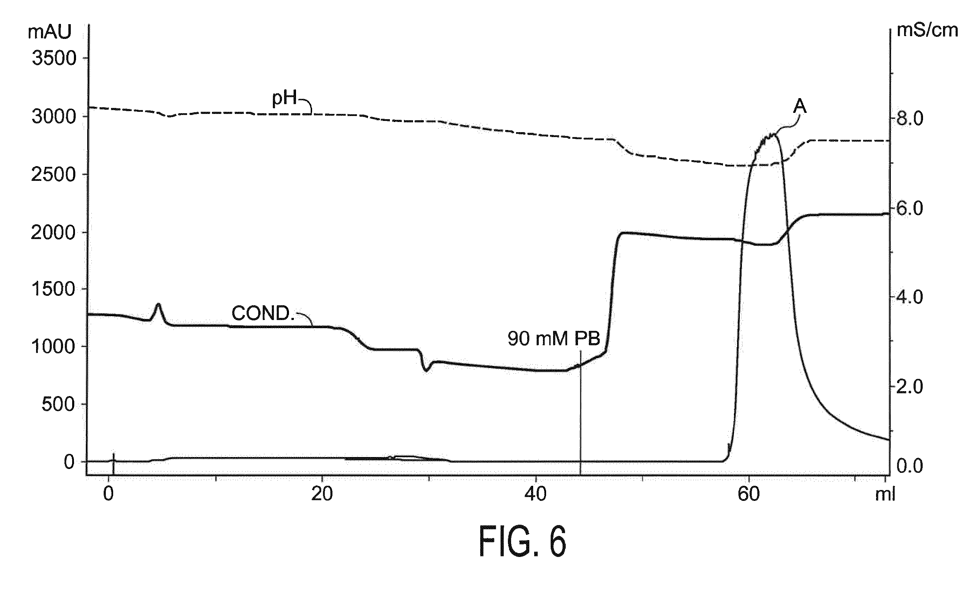

Figure 6 is an illustration of a chromatogram from the procedure described in this example. The line marked "Cond" represents the increase in conductivity in mS/cm. Peak A represents the eluate obtained from a step increase in conductivity from 35 mM buffer to 90 mM buffer.

### EXAMPLE 6

### Anion Exchange chromatography

The eluate obtained from cation exchange chromatography described in Example 5 was loaded onto an anion exchange resin (Q-Sepharose FF, VL32x250, 80 mL) pre-equilibrated with 5-20 CV of an equilibration buffer (67 mM phosphate buffer, pH 6). This was followed by a post load wash with 5 CV of equilibration buffer and the load and wash flow-through collected. Impurities levels in the cation exchange chromatography eluate and anion exchange chromatography flow-through are described in Table 2.

**Table 2**

| **Sample** | **Impurity Concentration** | |
|---|---|---|
| | **Host Cell Proteins** | **Protein A Leachates** |
| Clarified cell culture broth | 106551 ppm | **-** |
| Protein A eluate | 2.24 ppm | 12.96 ppm |
| Cation exchange chromatography (load pH 6) eluate | BDL | BDL |
| Anion exchange chromatography (load pH 6.0) | BDL | BDL |

| | | |
|---|---|---|
| BDL: Below detection limit. ppm: parts per million. | | |

There have been disclosed hereinbefore processes defined by the following numbered paragraphs:
1. A process for purifying antibodies comprising:
   a) purifying using protein A chromatography, wherein the antibody is eluted at a particular pH value; and
   b) purifying using cation exchange chromatography in the bind-elute mode, wherein an eluate from step a) is loaded onto a cation exchange resin without substantial adjustment of pH.
2. A process according to paragraph 1, wherein the antibody is eluted in step a) at pH values about 3.3 to about 4.5.
3. A process according to paragraph 1, wherein the antibody is eluted in step a) at a pH value about 3.5.
4. A process for purifying antibodies, comprising:
   a) purifying using protein A chromatography, wherein an antibody is eluted at a first pH value;
   b) purifying using cation exchange chromatography performed in the bind-elute mode, wherein eluate obtained from step a) is loaded onto a cation exchange resin at a second pH value, and elution is carried out at a third pH value; and
   c) purifying using anion exchange chromatography performed in the flow-through mode, wherein eluate from step b) is loaded on to an anion exchange resin without substantial adjustment of pH.
5. A process according to paragraph 4, wherein the first and second pH values are similar.
6. A process according to paragraph 4, wherein the first and second values are different.
7. A process according to paragraph 4, wherein the second and third pH values are similar.
8. A process according to paragraph 4, wherein the first pH value is about 3.5
9. A process according to paragraph 4, wherein the second pH value is about 6 to about 8.
10. A process according to paragraph 4, wherein the second pH value is about 6.
11. A process according to paragraph 4, wherein the third pH value is about 6 to about 8.
12. A process according to paragraph 4, wherein the third pH value is about 6.
13. A process according to paragraph 4, wherein elution performed in step b uses a linear or a step-wise increase of eluting fluid conductivity.

## Claims

1. A process for purifying antibodies, comprising:
a) purifying using protein A chromatography, wherein an antibody is eluted at a first pH value;
b) purifying using cation exchange chromatography performed in the bind-elute mode, wherein eluate obtained from step a) is loaded onto a cation exchange resin at a second pH value, and elution is carried out at a third pH value; and
c) purifying using anion exchange chromatography performed in the flow-through mode, wherein eluate from step b) is loaded on to an anion exchange resin without substantial adjustment of pH.

2. A process according to claim 1, wherein the first and second pH values are similar.

3. A process according to claim 1, wherein the first and second values are different.

4. A process according to claim 1, wherein the second and third pH values are similar.

5. A process according to claim 1, wherein the first pH value is about 3.5

6. A process according to claim 1, wherein the second pH value is about 6 to about 8.

7. A process according to claim 1, wherein the second pH value is about 6.

8. A process according to claim 1, wherein the third pH value is about 6 to about 8.

9. A process according to claim 1, wherein the third pH value is about 6.

10. A process according to claim 1, wherein elution performed in step b uses a linear or a step-wise increase of eluting fluid conductivity.
